(19) Europäisches Patentamt
European Patent Office
Office européen des brevets

(11) EP 3 932 900 A1

(12) EUROPEAN PATENT APPLICATION
published in accordance with Art. 153(4) EPC

(43) Date of publication:
**05.01.2022 Bulletin 2022/01**

(21) Application number: **20763187.0**

(22) Date of filing: **25.02.2020**

(51) Int Cl.:
***C07C 69/593*** *(2006.01)* ***C07C 67/30*** *(2006.01)*
***C07C 67/327*** *(2006.01)*

(86) International application number:
**PCT/JP2020/007286**

(87) International publication number:
**WO 2020/175420 (03.09.2020 Gazette 2020/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2019 JP 2019032640**

(71) Applicant: **Toray Industries, Inc.**
**Tokyo, 103-8666 (JP)**

(72) Inventors:
- **NAKAMURA, Hitomi**
  **Kamakura-shi, Kanagawa 248-8555 (JP)**
- **TSUKAMOTO, Daijiro**
  **Kamakura-shi, Kanagawa 248-8555 (JP)**
- **KAWAMURA, Kenji**
  **Kamakura-shi, Kanagawa 248-8555 (JP)**
- **YAMADA, Katsushige**
  **Kamakura-shi, Kanagawa 248-8555 (JP)**

(74) Representative: **Kador & Partner PartG mbB**
**Corneliusstraße 15**
**80469 München (DE)**

# (54) METHOD FOR PRODUCING ?,?-UNSATURATED DICARBOXYLIC ACID ESTER

(57) In a method for producing an α,β-unsaturated dicarboxylic acid ester exemplified by an α-hydromuconic acid ester from a carboxylic acid ester exemplified by a 3-hydroxyadipic acid ester or a 3-hydroxyadipic acid-3,6-lactone ester, the selectivity for the α,β-unsaturated dicarboxylic acid ester can be raised by subjecting the carboxylic acid ester to a basic condition of pH 8.5 to less than 13 in an organic solvent or a mixed solvent of an organic solvent and water.



Printed by Jouve, 75001 PARIS (FR)

## Description

### TECHNICAL FIELD

The present invention relates to a method of producing an α,β-unsaturated dicarboxylic acid ester.

### BACKGROUND ART

An α,β-unsaturated dicarboxylic acid ester is industrially useful as intermediates for syntheses, starting materials for medicines, starting materials for resins, etc. As methods of producing an α,β-unsaturated dicarboxylic acid ester which are generally conceived of from findings in organic synthesis chemistry, there are methods in which a carboxylic acid ester is subjected to basic conditions. For example, Non-Patent Document 1 discloses that 3-phenyl-3-hydroxyadipic acid-3,6-lactone ethyl ester is stirred in a mixed solvent of water and methanol under basic conditions with a pH of 13.9, thereby yielding 3-phenyl-α-hydromuconic acid, which is an α,β-unsaturated dicarboxylic acid. Non-Patent Document 2 discloses that 3-methyl-3-hydroxyadipic acid-3,6-lactone ethyl ester is stirred in water under basic conditions with a pH of 14 or higher, thereby yielding 3-methyl-α-hydromuconic acid as an α,β-unsaturated dicarboxylic acid.

Furthermore, Patent Document 1 discloses a method of producing an α,β-unsaturated carboxylic acid ester by preparing an aqueous solution containing a 3-hydroxycarboxylic acid ester, an alcohol solvent, and a dehydration catalyst and heating the reaction solution at a high temperature.

### CITATION LIST

### PATENT LITERATURE

Patent Document 1: JP-A-2015-187129

### NON-PATENT LITERATURE

Non-Patent Document 1: Journal of Chemical Society, pp. 4426-4428 (1956)
Non-Patent Document 2: Journal of Chemical Society C: Organic, issue 22, pp. 2314-2316 (1967)

### SUMMARY OF THE INVENTION

### TECHNICAL PROBLEMS

As a result of investigations made by the present inventors, a problem has newly been found out in which under the basic conditions shown in Non-Patent Document 1 and 2, an α,β-unsaturated dicarboxylic acid ester is hardly yielded from a carboxylic acid ester represented by general formula (I) or (II) below.

Furthermore, although there is a possibility that the method described in Patent Document 1 might be capable of selectively producing an α,β-unsaturated dicarboxylic acid ester from a carboxylic acid ester represented by general formula (I) or (II) below, this method is disadvantageous from the standpoint of economic efficiency because the reaction solution needs to be heated to a high temperature of 200°C or above.

Accordingly, an object of the present invention is to provide an economical method of selectively producing an α,β-unsaturated dicarboxylic acid ester from one or more carboxylic acid esters represented by the following general formula (I) and/or (II).

[Chem. 1]

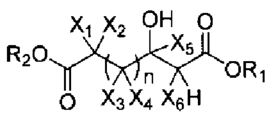

(I)

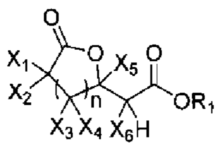

(II)

[In the formulae, n is an integer of 1-3, $X_1$ to $X_6$ each independently represent a hydrogen atom (H), an alkyl group having 1-6 carbon atoms, or a phenyl group, and $R_1$ and $R_2$ each independently represent an alkyl group having 1-6 carbon atoms.]

SOLUTION TO THE PROBLEMS

The present inventors diligently made investigations in order to overcome the problems and, as a result, have discovered that an α,β-unsaturated dicarboxylic acid ester can be economically produced with high selectively by subjecting one or more carboxylic acid esters represented by general formula (I) and/or general formula (II), as a starting material, to a basic condition with a pH less than 13 in either an organic solvent or a mixed solvent including an organic solvent and water. The present invention has been thus completed.

Specifically, the present invention is as shown below under (1) to (5).

(1) A method of producing an α,β-unsaturated dicarboxylic acid ester represented by general formula (III), the method including a step in which one or more carboxylic acid esters represented by general formula (I) and/or general formula (II) are subjected to a basic condition with pH of 8.5 or higher but less than 13 in either an organic solvent or a mixed solvent including an organic solvent and water.

[Chem. 2]

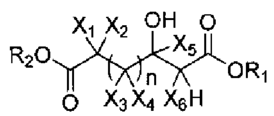

(I)

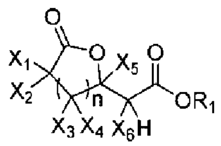

(II)

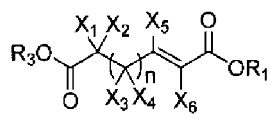

(III)

[In which, n is an integer of 1-3, $X_1$ to $X_6$ each independently represent a hydrogen atom (H), an alkyl group having 1-6 carbon atoms, or a phenyl group, $R_1$ and $R_2$ each independently represent an alkyl group having 1-6 carbon atoms, and $R_3$ represents a hydrogen atom (H) or an alkyl group having 1-6 carbon atoms.]

(2) The method according to (1), in which the organic solvent is a water-miscible organic solvent.

(3) The method according to (1) or (2), in which the mixed solvent including an organic solvent and water has a water content of 90% by volume or less.

(4) The method according to any of (1) to (3), in which the carboxylic acid ester represented by general formula (I) is a 3-hydroxyadipic acid ester.

(5) The method according to any one of (1) to (3), in which the carboxylic acid ester represented by general formula (II) is a 3-hydroxyadipic acid-3,6-lactone ester.

ADVANTAGEOUS EFFECTS OF THE INVENTION

According to the present invention, an α,β-unsaturated dicarboxylic acid ester can be economically produced with high selectivity by the method of producing the α,β-unsaturated dicarboxylic acid ester from one or more carboxylic acid esters represented by general formula (I) and/or general formula (II) as a starting material.

DESCRIPTION OF EMBODIMENTS

The present invention is explained below in more detail.

[Starting Material]

In the present invention, one or more carboxylic acid esters represented by the following general formula (I) and/or general formula (II) are used as a starting material.

[Chem. 3]

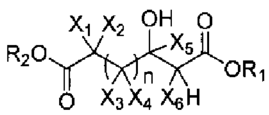

(I)

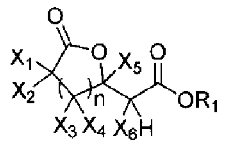

(II)

[In the formulae, n is an integer of 1-3, $X_1$ to $X_6$ each independently represent a hydrogen atom (H), an alkyl group having 1-6 carbon atoms, or a phenyl group, and $R_1$ and $R_2$ each independently represent an alkyl group having 1-6 carbon atoms.]

Symbol n in general formulae (I) and (II) is preferably 1.

It is preferable that $X_1$ to $X_6$ in general formulae (I) and (II) are each independently a hydrogen atom (H), an alkyl group having 1-2 carbon atoms, or a phenyl group. It is more preferable that $X_1$ to $X_4$ and $X_5$ are each a hydrogen atom (H), an alkyl group having 1-2 carbon atoms (methyl or ethyl group), or a phenyl group and $X_6$ is a hydrogen atom (H). It is still more preferable that $X_1$ to $X_6$ are all hydrogen atoms (H). That is, the carboxylic acid ester represented by general formula (I) is more preferably 3-hydroxyadipic acid ester, and the carboxylic acid ester represented by general formula (II) is more preferably 3-hydroxyadipic acid-3,6-lactone ester. The alkyl group having 1-2 carbon atoms is preferably a methyl group.

It is preferable that $R_1$ and $R_2$ in general formulae (I) and (II) are each independently an alkyl group having 1-3 carbon atoms (methyl, ethyl, or propyl group).

Preferred specific examples of the carboxylic acid esters represented by general formulae (I) and (II) respectively include the carboxylic acid diesters represented by the following formulae (1-1) to (1-27) and the carboxylic acid lactone esters represented by the following formulae (II-1) to (II-9). Of these, preferred carboxylic acid esters represented by general formula (I) are the 3-hydroxyadipic acid esters represented by the following formulae (1-1) to (1-9), and more preferred is dimethyl 3-hydroxyadipate, which is represented by formula (1-1). Preferred carboxylic acid esters represented by general formula (II) are the 3-hydroxyadipic acid-3,6-lactone esters represented by the following formulae (II-1) to (II-3), and more preferred is 3-hydroxyadipic acid-3,6-lactone methyl ester, which is represented by formula (II-1).

[Chem. 4]

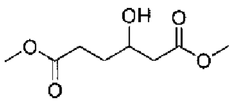
(I-1)

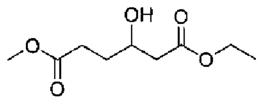
(I-2)

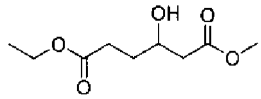
(I-3)

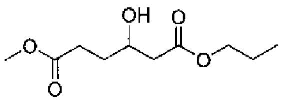
(I-4)

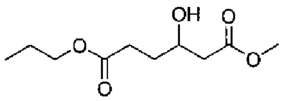
(I-5)

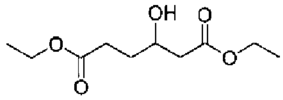
(I-6)

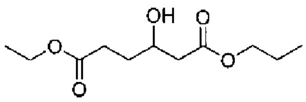
(I-7)

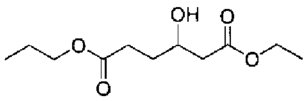
(I-8)

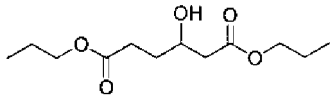
(I-9)

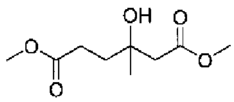
(I-10)

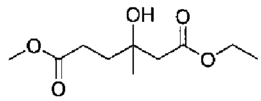
(I-11)

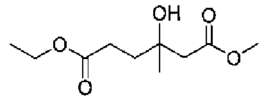
(I-12)

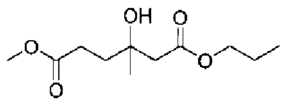
(I-13)

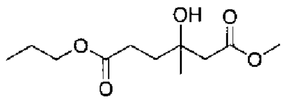
(I-14)

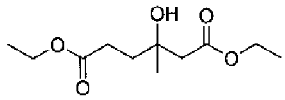
(I-15)

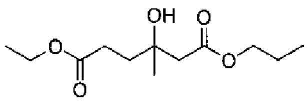
(I-16)

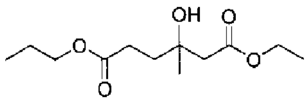
(I-17)

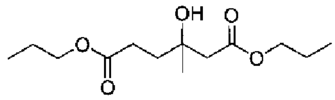
(I-18)

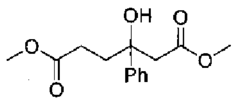
(I-19)

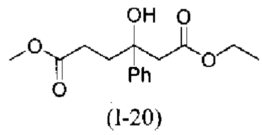
(I-20)

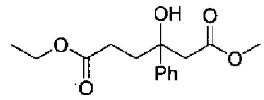
(I-21)

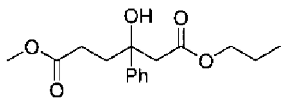
(I-22)

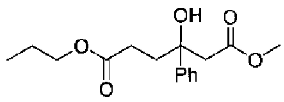
(I-23)

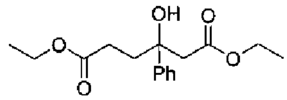
(I-24)

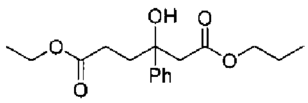
(I-25)

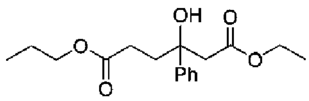
(I-26)

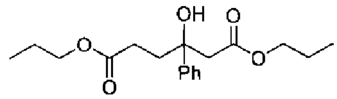
(I-27)

[Chem. 5]

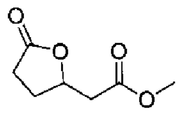
(II-1)

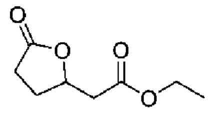
(II-2)

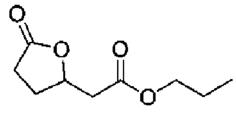
(II-3)

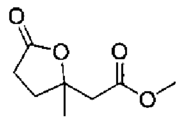
(II-4)

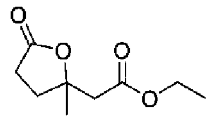
(II-5)

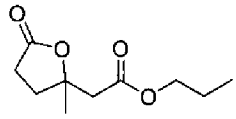
(II-6)

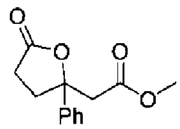
(II-7)

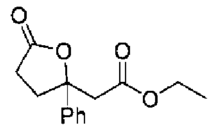
(II-8)

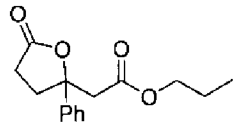
(II-9)

The carboxylic acid esters represented by general formula (I) or (II) to be used as a starting material in the present invention can be either chemically synthesized products or ones derived from renewable biomass resources.

As methods of obtaining carboxylic acid esters usable as a starting material in the present invention by chemical syntheses, in the case of dimethyl 3-hydroxyadipate which is represented by formula (1-1), or 3-hydroxyadipic acid-3,6-lactone methyl ester which is represented by formula (II-1), the ester can be produced by esterifying 3-hydroxyadipic acid or 3-hydroxyadipic acid-3,6-lactone (as shown in Reference Examples 5 and 6 in this description). Furthermore, 3-hydroxyadipic acid-3,6-lactone methyl ester (II-1), for example, can be obtained by treating dimethyl 3-hydroxyadipate (1-1) with an acid.

For synthesizing a carboxylic acid ester represented by general formula (I) or (II) to be used as a starting material in the present invention from biomass resources, the following method can be used. In the case of dimethyl 3-hydroxyadipate which is represented by formula (1-1), or 3-hydroxyadipic acid-3,6-lactone methyl ester which is represented by formula (II-1), 3-hydroxyadipic acid is produced from biomass resources by microbial fermentation by the method described in International Publication WO 2016/199856 and the 3-hydroxyadipic acid is esterified, thereby producing the desired ester (as shown in Reference Example 5 in this description). The 3-hydroxyadipic acid may be isolated from a fermentation broth containing 3-hydroxyadipic acid obtained by microbial fermentation. Alternatively, the fermentation broth containing 3-hydroxyadipic acid may be subjected as such to esterification.

Methods for esterifying carboxylic acids which are starting materials for carboxylic acid esters represented by general formula (I) or (II) and have the same backbones as the carboxylic acid esters represented by general formula (I) or (II) are not particularly limited. Examples thereof include an esterification reaction in which an acid catalyst and an alcohol solvent are used. The acid catalyst to be used here is not particularly limited, and examples thereof include mineral acids such as sulfuric acid and hydrochloric acid and solid acids such as silica and strong acid resins. Other examples of methods of carboxylic-acid esterification include: dehydrating condensation of an alcohol with the carboxylic acid in which a condensing agent is used; dehydrating condensation of an alcohol with the carboxylic acid in which a Lewis acid such as a boron trifluoride-methanol complex is used; a method of production under basic conditions in which a metal alkoxide is used; and a production method in which an alkylation reagent such as diazomethane or an alkyl halide is used.

[$\alpha$,$\beta$-Unsaturated Dicarboxylic Acid Ester]

The $\alpha$,$\beta$-unsaturated dicarboxylic acid ester which can be produced by the present invention is an $\alpha$,$\beta$-unsaturated dicarboxylic acid ester represented by the following general formula (III). The $\alpha$,$\beta$-unsaturated dicarboxylic acid ester to be obtained may be a cis isomer alone or a trans isomer alone or may be a mixture of a cis isomer and a trans isomer. In the present invention, a trans isomer alone can be preferably produced.

[Chem. 6]

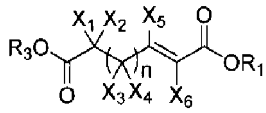

(III)

[In the formula, n is an integer of 1-3, $X_1$ to $X_6$ each independently represent a hydrogen atom (H), an alkyl group having 1-6 carbon atoms, or a phenyl group, $R_1$ represents an alkyl group having 1-6 carbon atoms, and $R_3$ represents a hydrogen atom (H) or an alkyl group having 1-6 carbon atoms.]

As in general formula (I) or (II) described above, n in general formula (III) is preferably 1.

Similarly, it is preferable that $X_1$ to $X_6$ in general formula (III) are each independently a hydrogen atom (H), an alkyl group having 1-2 carbon atoms, or a phenyl group. It is more preferable that $X_1$ to $X_4$ and $X_5$ are each a hydrogen atom (H), an alkyl group having 1-2 carbon atoms (methyl or ethyl group), or a phenyl group and $X_6$ is a hydrogen atom (H). It is still more preferable that $X_1$ to $X_6$ are all hydrogen atoms (H). That is, the α,β-unsaturated carboxylic acid ester represented by general formula (III) is more preferably an α-hydromuconic acid ester. The alkyl group having 1-2 carbon atoms is preferably a methyl group.

Similarly, $R_1$ in general formula (III) is preferably an alkyl group having 1-3 carbon atoms (methyl, ethyl, or propyl group).

Furthermore, $R_3$ in general formula (III) is preferably a hydrogen atom (H) or an alkyl group having 1-3 carbon atoms (methyl, ethyl, or propyl group).

Preferred specific examples of the α,β-unsaturated carboxylic acid ester represented by general formula (III) include the monoesters of α,β-unsaturated dicarboxylic acids represented by the following formulae (III-1) to (III-9) and the diesters of α,β-unsaturated dicarboxylic acids represented by the following formulae (III-10) to (III-36), the monoesters and the diesters being obtained using the carboxylic acid diesters represented by formulae (I-1) to (1-27) shown above and/or the carboxylic acid lactone esters represented by formulae (II-1) to (II-9) shown above, as starting materials. Preferred of these are the α-hydromuconic acid monoesters represented by the following formulae (III-1) to (III-3) or the α-hydromuconic acid diesters represented by the following formulae (III-10) to (III-18), these monoesters and diesters being obtained when the 3-hydroxyadipic acid esters represented by formulae (1-1) to (1-9) and/or the 3-hydroxyadipic acid-3,6-lactone esters represented by formulae (II-1) to (II-3) are used as starting materials. More preferred are monomethyl α-hydromuconate, which is represented by the following formula (III-1), or dimethyl α-hydromuconate, which is represented by the following formula (III-10), these esters being obtained when dimethyl 3-hydroxyadipate, which is represented by formula (I-1), and/or 3-hydroxyadipic acid-3,6-lactone methyl ester, which is represented by formula (II-1), is used as a starting material.

[Chem. 7]

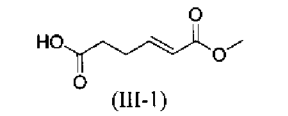
(III-1)
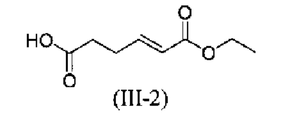
(III-2)
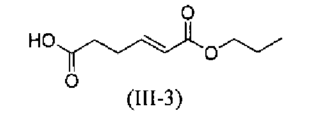
(III-3)
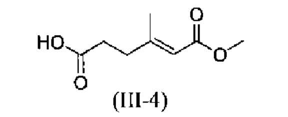
(III-4)
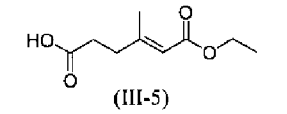
(III-5)
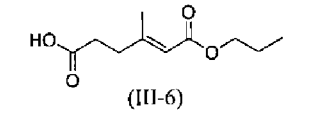
(III-6)
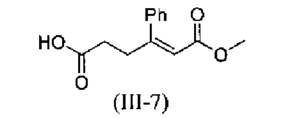
(III-7)
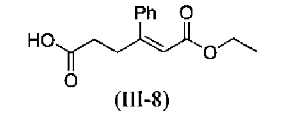
(III-8)
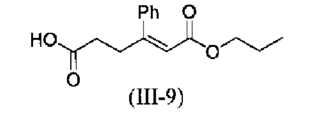
(III-9)
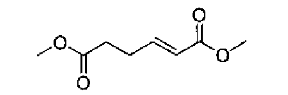
(III-10)
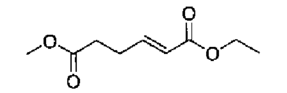
(III-11)
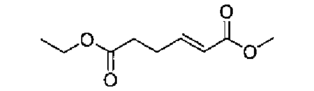
(III-12)
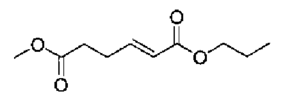
(III-13)
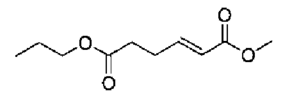
(III-14)
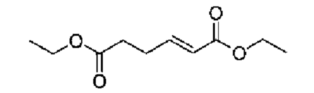
(III-15)
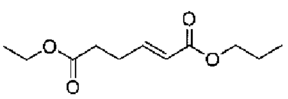
(III-16)
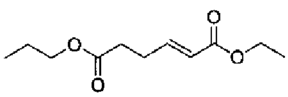
(III-17)
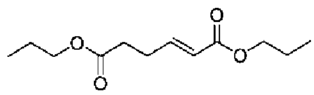
(III-18)
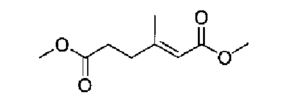
(III-19)
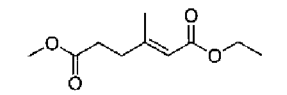
(III-20)
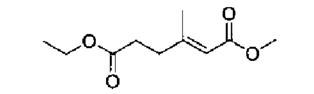
(III-21)
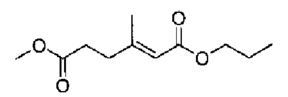
(III-22)
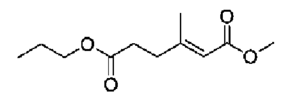
(III-23)
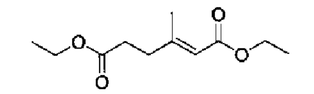
(III-24)
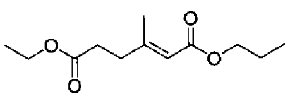
(III-25)
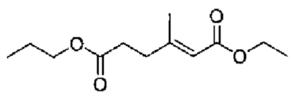
(III-26)
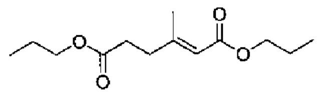
(III-27)
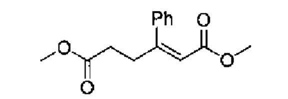
(III-28)
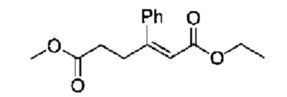
(III-29)
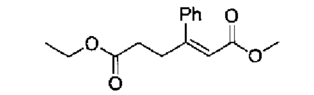
(III-30)
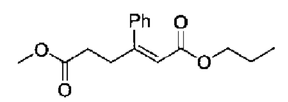
(III-31)
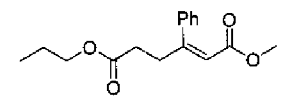
(III-32)
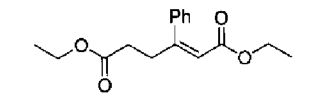
(III-33)
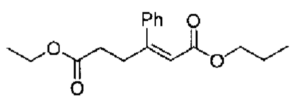
(III-34)
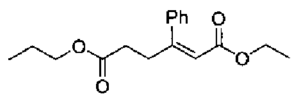
(III-35)
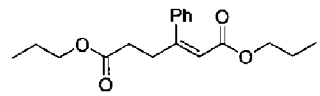
(III-36)

In the case where the α,β-unsaturated carboxylic acid ester to be obtained by the present invention is a monoester, the desired product is obtained as a salt of the α,β-unsaturated carboxylic acid monoester. Specific examples of the α,β-unsaturated carboxylic acid monoester salt include sodium salts, potassium salts, lithium salts, magnesium salts, calcium salts, and ammonium salts. In this description, an α,β-unsaturated carboxylic acid monoester, regardless

of whether it is in its free form or in a salt form, is referred to simply as "α,β-unsaturated carboxylic acid monoester".

[Reaction Solvent]

In the method of producing an α,β-unsaturated dicarboxylic acid ester of the present invention, either an organic solvent or a mixed solvent including an organic solvent and water is used as a reaction solvent.

The organic solvent to be used in the method of producing an α,β-unsaturated dicarboxylic acid ester of the present invention is not particularly limited. However, it is preferably a water-miscible organic solvent. The term "water-miscible organic solvent" means an organic solvent which can be mixed with water in any proportion. Examples of the water-miscible organic solvent include methanol, ethanol, n-propanol, isopropanol, tert-butyl alcohol, ethylene glycol, 1,2-dimethoxyethane, acetone, tetrahydrofuran, acetonitrile, dimethyl sulfoxide, dioxane, and dimethylformamide. One of these water-miscible organic solvents can be used alone, or a mixed solvent composed of two or more of these may be used. From an industrial standpoint, it is preferred to use an organic solvent in which the starting material according of the present invention dissolves satisfactorily. Preferred of those water-miscible organic solvents is methanol, ethanol, n-propanol, isopropanol, acetone, tetrahydrofuran, dioxane, or a mixed solvent composed of two or more of these. More preferred is a mixed solvent composed of acetone and methanol.

In the case where a mixed solvent composed of an organic solvent and water is used as the reaction solvent, the mixed solvent is preferably one composed of a water-miscible organic solvent and water. More preferred is a mixed solvent composed of water and methanol, ethanol, n-propanol, isopropanol, acetone, tetrahydrofuran, dioxane, or a mixed solvent composed of two or more of these. Still more preferred is a mixed solvent composed of water and either methanol or acetone. From the standpoint of stably regulating the basic conditions during reaction which will be described later, the reaction solvent is preferably a solvent including water or methanol in an amount of 10% by volume or larger. The water content in the mixed solvent composed of an organic solvent and water is not particularly limited, but is usually 90% by volume or less, preferably 80% by volume of less, more preferably 70% by volume of less, still more preferably 50% by volume or less, yet still more preferably 10-80% by volume, especially preferably 10-50% by volume.

[Basic Conditions]

In the method of producing an α,β-unsaturated dicarboxylic acid ester of the present invention, the carboxylic acid ester is subjected, in the reaction solvent, to basic conditions with a pH of 8.5 or higher but less than 13. The values of pH herein are ones measured with a pH meter in common use. By performing the reaction under such pH conditions, the selectivity to the α,β-unsaturated carboxylic acid ester can be heightened. Preferred pH conditions include a pH of 10.0 or higher but less than 12.5.

Bases usable for preparing the basic conditions are not particularly limited so long as the pH of the reaction solution can be regulated therewith. An inorganic base or an organic base can be used.

Examples of the inorganic base include hydroxides, carbonates, and hydrides. More specific examples include lithium hydroxide, sodium hydroxide, potassium hydroxide, calcium hydroxide, magnesium hydroxide, ammonium hydroxide, sodium hydrogen carbonate, potassium hydrogen carbonate, ammonium hydrogen carbonate, sodium carbonate, potassium carbonate, ammonium carbonate, lithium hydride, sodium hydride, and potassium hydride. Preferred of these are lithium hydroxide, sodium hydroxide, potassium hydroxide, calcium hydroxide, magnesium hydroxide, ammonium hydroxide, sodium hydrogen carbonate, potassium hydrogen carbonate, ammonium hydrogen carbonate, sodium carbonate, potassium carbonate, ammonium carbonate, and sodium hydride. More preferred are lithium hydroxide, sodium hydroxide, potassium hydroxide, sodium hydrogen carbonate, potassium hydrogen carbonate, sodium carbonate, and potassium carbonate.

Examples of the organic base include alkoxide bases, ammonium salts, and amine-compound bases. Specific examples include sodium methoxide, sodium ethoxide, sodium n-propanolate, sodium 2-propanolate, sodium tert-butoxide, sodium phenoxide, potassium methoxide, potassium ethoxide, potassium n-propanolate, potassium 2-propanolate, potassium tert-butoxide, tetramethylammonium hydroxide, tetrabutylammonium hydroxide, trimethylethanolammonium hydroxide, triethylamine, N,N-diisopropylethylamine, pyridine, aniline, imidazole, benzimidazole, histidine, guanidine, piperidine, pyrrolidine, morpholine, diazabicycloundecene, and diazabicyclononene. Preferred of these are sodium methoxide, sodium ethoxide, sodium n-propanolate, sodium 2-propanolate, sodium tert-butoxide, potassium methoxide, potassium ethoxide, potassium n-propanolate, potassium 2-propanolate, potassium tert-butoxide, tetrabutylammonium hydroxide, triethylamine, N,N-diisopropylethylamine, pyridine, imidazole, histidine, guanidine, diazabicycloundecene, and diazabicyclononene. More preferred are sodium methoxide, sodium ethoxide, sodium tert-butoxide, potassium methoxide, potassium ethoxide, potassium tert-butoxide, triethylamine, N,N-diisopropylethylamine, pyridine, diazabicycloundecene, and diazabicyclononene.

One of those bases may be used alone as the base for preparing the basic conditions, or two or more of those bases may be used as a mixture thereof for preparing the basic conditions.

The amount of the base(s) to be added to a reaction solution or a reaction solvent in order to prepare the basic conditions is not particularly limited so long as the reaction solution is kept under basic conditions with a pH of 8.5 or higher but less than 13.

The pH of the reaction solution may be regulated by regulating the pH of the reaction solvent before addition of the starting material, or may be regulated after the starting material has been added to the reaction solvent. In cases when the pH of the reaction solution decreases as the conversion of the carboxylic acid ester represented by general formula (I) or (II) into an α,β-unsaturated dicarboxylic acid ester proceeds, a base may be suitably supplemented during the reaction in order to keep the pH of the reaction solution within the adequate basic conditions.

[Reaction Temperature]

In the present invention, the reaction temperature in producing an α,β-unsaturated dicarboxylic acid ester is not particularly limited. However, the reaction temperature is preferably 0°C or higher and 200°C or lower, more preferably 10°C or higher and 100°C or lower, still more preferably 15°C or higher and 80°C or lower.

[Reaction Pressure]

The reaction pressure in producing an α,β-unsaturated dicarboxylic acid ester in the present invention is not particularly limited. The reaction pressure is preferably 0.01 MPa or higher and 0.5 MPa or lower. In particular, it is easy to conduct the reaction at the atmospheric pressure because no device or operation for depressurization or pressurization is necessary.

[Esterification of α,β-Unsaturated Dicarboxylic Acid Monoester]

In the case where the α,β-unsaturated dicarboxylic acid ester obtained in the present invention is an α,β-unsaturated dicarboxylic acid monoester such as, for example, any of those represented by formulae (III-1) to (III-9), this monoester can be converted to an α,β-unsaturated dicarboxylic acid diester by further subjecting the monoester to an esterification reaction. Methods for the esterification are not particularly limited, and examples thereof include an esterification reaction in which an acid catalyst and an alcohol solvent are used. The acid catalyst to be used here is not particularly limited, and examples thereof include mineral acids such as sulfuric acid and hydrochloric acid and solid acids such as silica and strong acid resins. Other examples of methods for carboxylic-acid esterification include: dehydrating condensation of an alcohol with the carboxylic acid in which a condensing agent is used; dehydrating condensation of an alcohol with the carboxylic acid in which a Lewis acid such as a boron trifluoride-methanol complex is used; a method of production under basic conditions in which a metal alkoxide is used; and a production method in which an alkylation reagent such as diazomethane or an alkyl halide is used.

[Modes of Reaction]

The method of producing an α,β-unsaturated dicarboxylic acid ester of the present invention can be conducted in a mode using a reactor which is any of a batch vessel reactor, a semi-batch vessel reactor, a continuous-process vessel reactor, and a continuous-process tubular reactor.

[Recovery of the α,β-Unsaturated Dicarboxylic Acid Ester]

The α,β-unsaturated dicarboxylic acid ester obtained in the present invention can be recovered after completion of the reaction by an ordinary separation and purification operation such as solid/liquid separation by filtration, crystallization, extraction, distillation, or adsorption.

EXAMPLES

The present invention is explained below in more detail by reference to Examples, but the present invention is not limited to the following Examples. Reaction results in the Reference Examples, Examples, and Comparative Examples are defined by the following expressions.

$$\text{Conversion of starting material (\%)} = [(\text{supplied starting material (mol)}) - (\text{unreacted starting material (mol)})]/(\text{supplied starting material (mol)}) \times 100$$

$$\text{Product selectivity (\%)} = (\text{amount of yielded product (mol)})/[(\text{supplied starting material (mol)}) - (\text{unreacted starting material (mol)})] \times 100$$

Each reaction solution was analyzed by high-performance liquid chromatography (HPLC). The amount of the product was determined with an absolute calibration curve drawn using standard samples. Conditions for the analysis by HPLC are as follows.

[HPLC Analysis Conditions]

HPLC apparatus: Prominence (manufactured by Shimadzu Corp.)
Column: Synergi hydro-RP (manufactured by Phenomenex Inc.); length, 250 mm; inner diameter, 4.60 mm; particle diameter, 4 μm

Mobile phase: 0.1 wt% aqueous phosphoric acid solution/acetonitrile = 95/5 (volume ratio; 10-minute holding) → 80/20 (gradient, 10 minutes) → 30/70 (gradient, 6 minutes) → 95/5 (gradient, 3 minutes; 11-minute holding)

Flow rate: 1.0 mL/min
Detector: UV (210 nm)
Column temperature: 40°C

The pH of reaction solutions and reaction solvents were analyzed by the following method.

[Method of analyzing the pH of Reaction Solution and Reaction Solvent]

pH meter: Horiba pH Meter F-52 (manufactured by Horiba Ltd.)
Reference solutions used for calibration: pH 7 (neutral phosphoric acid salt; pH 6.86 at 25°C), pH 4 (phthalic acid salt; pH 4.01 at 25°C), pH 9 (boric acid salt; 9.18 at 25°C)
Calibration method: the pH-7 reference solution was used to determine a zero point and the pH-4 reference solution and the pH-9 reference solution were subsequently used to perform three-point calibration.

The 3-hydroxyadipic acid, 3-hydroxyadipic acid-3,6-lactone, and α-hydromuconic acid used in the following Reference Examples 1 to 5 had been produced by the methods described in International Publication WO 2016/068108.

(Reference Example 1) Preparation of Dimethyl 3-Hydroxyadipate (1-1)

Dimethyl 3-hydroxyadipate, which was used as a starting material and as a standard sample for HPLC analysis in Examples, was prepared by chemical synthesis. To 10.0 g (0.06 mol) of 3-hydroxyadipic acid was added 100 mL of anhydrous methanol (manufactured by FUJIFILM Wako Pure Chemical Corp.). Five drops of concentrated sulfuric acid (manufactured by FUJIFILM Wako Pure Chemical Corp.) were added thereto with stirring, and the resultant mixture was refluxed at 70°C for 5 hours. After completion of the reaction, the reaction mixture was concentrated with a rotary evaporator and then separated and purified by silica gel column chromatography (hexane/ethyl acetate = 4/1), thereby obtaining 5.6 g of pure dimethyl 3-hydroxyadipate (yield, 49%). An NMR spectrum of the obtained dimethyl 3-hydroxyadipate is as follows.
$^1$H-NMR (400 MHz, $CDCl_3$): δ 1.61-1.84 (m, 2H), δ 2.42-2.56 (m 4H), δ 3.10 (d, 1H), δ 3.69 (s, 3H), δ 3.72 (s, 3H), δ 4.02-4.07 (m, 1H).

(Reference Example 2) Preparation of 3-Hydroxyadipic acid-3,6-lactone Methyl Ester (II-1)

3-Hydroxyadipic acid-3,6-lactone methyl ester, which was used as a starting material and as a standard sample for HPLC analysis in Examples and Comparative Examples, was prepared by chemical synthesis. To 10.0 g (0.06 mol)

of 3-hydroxyadipic acid was added 100 mL of anhydrous methanol (manufactured by FUJIFILM Wako Pure Chemical Corp.). Five drops of concentrated sulfuric acid (manufactured by FUJIFILM Wako Pure Chemical Corp.) were added thereto with stirring, and the resultant mixture was refluxed at 70°C for 5 hours. After completion of the reaction, the reaction mixture was concentrated with a rotary evaporator and then separated and purified by silica gel column chromatography (hexane/ethyl acetate = 4/1), thereby obtaining 5.4 g of pure 3-hydroxyadipic acid-3,6-lactone methyl ester (yield, 48%). An NMR spectrum of the obtained 3-hydroxyadipic acid-3,6-lactone methyl ester is as follows.

$^1$H-NMR (400 MHz, $CDCl_3$): δ 1.93-2.02 (m, 1H), δ 2.44-2.52 (m, 1H), δ 2.56-2.87 (m, 2H), δ 2.66 (dd, 1H), δ 2.85 (dd, 1H), δ 3.73 (s, 3H), δ 4.87-4.94 (m, 1H).

(Reference Example 3) Preparation of Monomethyl α-Hydromuconate (III-1)

Monomethyl α-hydromuconate, which was used as a starting material and as a standard sample for HPLC in Examples, was prepared by chemical analysis. In 4.5 mL of methanol was dissolved 50 mg (0.35 mmol) of 3-hydroxyadipic acid-3,6-lactone methyl ester (II-1). Thereto was added 0.5 mL of 0.5-M aqueous sodium hydrogen carbonate solution. The resultant mixture was refluxed at 70°C for 8 hours. After completion of the reaction, the reaction mixture was concentrated with a rotary evaporator. The concentrate was dissolved in 10 mL of water, and 5 mL of 2-M aqueous hydrochloric acid solution was added thereto. This mixture was subjected to extraction with ethyl acetate. The recovered organic solvent was dehydrated with sodium sulfate and concentrated with a rotary evaporator, thereby obtaining 47 mg of pure monomethyl α-hydromuconate (yield, 94%). An NMR spectrum of the obtained monomethyl α-hydromuconate is as follows.

$^1$H-NMR (400 MHz, $CDCl_3$): δ 2.47 (s, 4H), δ 3.66 (s, 3H), δ 5.81 (d, 1H), δ 6.91 (dt, 1H), δ 10.65 (s, 1H).

(Reference Example 4) Preparation of Dimethyl α-Hydromuconate (III-10)

Dimethyl α-hydromuconate, which was used as a standard sample for HPLC analysis in Examples, was prepared by chemical synthesis. One gram (6.9 mmol) of a-hydromuconic acid was dissolved in 10 mL of methanol (manufactured by FUJIFILM Wako Pure Chemical Corp.), and two drops of concentrated sulfuric acid (manufactured by FUJIFILM Wako Pure Chemical Corp.) were added thereto. The resultant mixture was refluxed at 70°C for 6 hours. After completion of the reaction, the reaction mixture was concentrated with a rotary evaporator and purified by silica gel column chromatography (hexane/ethyl acetate = 7/3), thereby obtaining 0.9 g of pure dimethyl α-hydromuconate (yield, 75%). An NMR spectrum of the obtained dimethyl α-hydromuconate is as follows.

$^1$H-NMR (400 MHz, $CDCl_3$): δ 2.46-2.57 (m, 4H), δ 3.69 (s, 3H), δ 3.73 (s, 3H), δ 5.86 (d, 1H), δ 6.95 (dt, 1H).

(Reference Example 5) Production of Dimethyl 3-Hydroxyadipate (1-1) and 3-Hydroxyadipic acid-3,6-lactone Methyl Ester (II-1) from 3-Hydroxyadipic Acid

Ten milligrams of 3-hydroxyadipic acid and 9 mL of methanol (manufactured by FUJIFILM Wako Pure Chemical Corp.) were used and introduced into a round-bottom flask having a capacity of 25 mL (manufactured by IWAKI, AGC TECHNO GLASS Co., Ltd.), and 1 mL of 1-M aqueous sulfuric acid solution (manufactured by Nacalai Tesque, Inc.) was added as a catalyst thereto. This reaction solution was refluxed for 5 hours with stirring at 300 rpm and then recovered. A 0.1-mL portion of the reaction solution was diluted with 0.9 mL of water, filtered with a 0.22-μm filter, and then analyzed by HPLC. Yield of dimethyl 3-hydroxyadipate was 49%, and yield of 3-hydroxyadipic acid-3,6-lactone methyl ester was 42%.

(Reference Example 6) Production of Dimethyl 3-Hydroxyadipate (1-1) and 3-Hydroxyadipic acid-3,6-lactone Methyl Ester (II-1) from 3-Hydroxyadipic acid-3,6-lactone

A reaction was conducted in the same manner as in Reference Example 5, except that 10 mg of 3-hydroxyadipic acid-3,6-lactone was used as a starting material in place of the 3-hydroxyadipic acid. Yield of dimethyl 3-hydroxyadipate was 45%, and yield of 3-hydroxyadipic acid-3,6-lactone methyl ester was 51%.

(Example 1) Production of Monomethyl α-Hydromuconate (III-1) from Dimethyl 3-Hydroxyadipate (1-1) as Starting Material

One milligram of dimethyl 3-hydroxyadipate and 0.9 mL of methanol were used and introduced into a vial made of glass having a capacity of 2 mL (manufactured by LABORAN), and 0.1 mL of 0.1-M aqueous sodium hydrogen

carbonate solution was added thereto. The used 0.1-M aqueous sodium hydrogen carbonate solution was one obtained by dissolving 0.84 g of sodium hydrogen carbonate (manufactured by Nacalai Tesque, Inc.) in 100 mL of water. The resultant reaction solution was reacted at room temperature for 16 hours with stirring at 300 rpm and then recovered. The pH of the reaction solution during the reaction was 11.9-12.1. A 0.1-mL portion of the reaction solution after the reaction was diluted with 0.9 mL of water, filtered with a 0.22-μm filter, and then analyzed by HPLC. The results are shown in Table 1.

(Example 2) Production of Monomethyl α-Hydromuconate (III-1) from Mixture of Dimethyl 3-Hydroxyadipate (1-1) and 3-Hydroxyadipic acid-3,6-lactone Methyl Ester (II-1) as Starting-material Compounds

A reaction was conducted in the same manner as in Example 1, except that 0.5 mg of dimethyl 3-hydroxyadipate and 0.5 mg of 3-hydroxyadipic acid-3,6-lactone methyl ester were used as starting materials. The pH of the reaction solution during the reaction was 11.9-12.0. The results are shown in Table 1.

(Example 3) Production of Monomethyl α-Hydromuconate (III-1) from 3-Hydroxyadipic acid-3,6-lactone Methyl Ester (II-1) as Starting-material Compound

A reaction was conducted in the same manner as in Example 1, except that 1.0 mg of 3-hydroxyadipic acid-3,6-lactone methyl ester was used as a starting material. The pH of the reaction solution during the reaction was 11.3. The results are shown in Table 1.

(Example 4)

A reaction was conducted in the same manner as in Example 3, except that 0.1 mL of 0.1-M aqueous sodium hydroxide solution was added in place of the 0.1-M aqueous sodium hydrogen carbonate solution. The used 0.1-M aqueous sodium hydroxide solution was one obtained by diluting 1-M aqueous sodium hydroxide solution (manufactured by Nacalai Tesque, Inc.) 10 times with water. The pH of the reaction solution during the reaction was 12.0-12.2. The results are shown in Table 1.

(Example 5)

A reaction was conducted in the same manner as in Example 3, except that 0.1 mL of 0.5-M aqueous sodium hydroxide solution was added in place of the 0.1-M aqueous sodium hydrogen carbonate solution. The used 0.5-M aqueous sodium hydroxide solution was one obtained by diluting 1-M aqueous sodium hydroxide solution (manufactured by Nacalai Tesque, Inc.) 2 times with water. The pH of the reaction solution during the reaction was 12.8-12.9. The results are shown in Table 1.

(Example 6)

A reaction was conducted in the same manner as in Example 3, except that 0.1 mL of 0.01-M aqueous sodium hydrogen carbonate solution was added in place of the 0.1-M aqueous sodium hydrogen carbonate solution. The used 0.01-M aqueous sodium hydrogen carbonate solution was one obtained by dissolving 84 mg of sodium hydrogen carbonate (manufactured by Nacalai Tesque, Inc.) in 100 mL of water. The pH of the reaction solution during the reaction was 8.6-11.0. The results are shown in Table 1.

(Example 7)

A reaction was conducted in the same manner as in Example 3, except that 0.9 mL of anhydrous methanol (manufactured by FUJIFILM Wako Pure Chemical Corp.) was used as a reaction solvent and 0.1 mL of a 0.1-M methanol solution of sodium hydroxide (solution obtained by dissolving 40 mg of sodium hydroxide in 10 mL of anhydrous methanol) was added as a catalyst. The pH of the reaction solution during the reaction was 12.3-12.5. The results are shown in Table 1.

(Example 8)

A reaction was conducted in the same manner as in Example 4, except that a mixed solvent composed of 0.5 mL of methanol and 0.4 mL of water was used in place of the 0.9 mL of methanol. The pH of the reaction solution during the reaction was 11.0-12.2. The results are shown in Table 1.

(Example 9)

A reaction was conducted in the same manner as in Example 4, except that a mixed solvent composed of 0.3 mL of methanol and 0.6 mL of water was used in place of the 0.9 mL of methanol. The pH of the reaction solution during the reaction was 10.5-12.3. The results are shown in Table 1.

(Example 10)

A reaction was conducted in the same manner as in Example 4, except that a mixed solvent composed of 0.1 mL of methanol and 0.8 mL of water was used in place of the 0.9 mL of methanol. The pH of the reaction solution during the reaction was 9.2-12.2. The results are shown in Table 1.

(Example 11)

A reaction was conducted in the same manner as in Example 3, except that a mixed solvent composed of 0.5 mL of acetone and 0.4 mL of water was used in place of the 0.9 mL of methanol. The pH of the reaction solution during the reaction was 10.3-10.6. The results are shown in Table 1.

(Example 12)

A reaction was conducted in the same manner as in Example 7, except that a mixed solvent composed of 0.5 mL of acetone and 0.4 mL of anhydrous methanol was used in place of the 0.9 mL of anhydrous methanol. The pH of the reaction solution during the reaction was 12.5-12.9. The results are shown in Table 1.

(Comparative Example 1)

A reaction was conducted in the same manner as in Example 4, except that 0.1 mL of 1.0-M aqueous sodium hydroxide solution (manufactured by Nacalai Tesque, Inc.) was added in place of the 0.1 mL of 0.1-M aqueous sodium hydroxide solution. The pH of the reaction solution during the reaction was 13.0-13.3. The results are shown in Table 1.

(Comparative Example 2)

A reaction was conducted in the same manner as in Example 3, except that 0.1 mL of 0.1-mM aqueous sodium hydrogen carbonate solution was added in place of the 0.1 mL of 0.1-M aqueous sodium hydrogen carbonate solution. The used 0.1-mM aqueous sodium hydrogen carbonate solution was one obtained by dissolving 84 mg of sodium hydrogen carbonate (manufactured by Nacalai Tesque, Inc.) in 100 mL of water and then diluting the solution 100 times with water. The pH of the reaction solution during the reaction was 7.6-8.0. The results are shown in Table 1.

(Comparative Example 3)

A reaction was conducted in the same manner as in Example 4, except that a mixed solvent composed of 0.5 mL of acetone and 0.4 mL of water was used in place of the 0.9 mL of methanol. The pH of the reaction solution during the reaction was 14.0-14.2. The results are shown in Table 1.

[Table 1]

| Reaction | Starting material | pH range | Solvent | Proportion of water to reaction solvent (vol%) | Conversion of starting material (%) | Product Selectivity (%) | | | | |
|---|---|---|---|---|---|---|---|---|---|---|
| | | | | | | HMAM | HMA | 3HAD | 3HALE | 3HA |
| Example 1 | 3HAD | 11.9-12.1 | methanol/ water | 10 | 100 | 96.3 | 0.6 | - | undetected | undetected |
| Example 2 | 3HAD + 3HALE | 11.9-12.0 | methanol/ water | 10 | 100 | 99.1 | 0.7 | - | - | undetected |
| Example 3 | 3HALE | 11.3 | methanol/ water | 10 | 100 | 99.5 | 0.5 | undetected | - | undetected |
| Example 4 | 3HALE | 12-12.2 | methanol/ water | 10 | 100 | 98 | 1 | undetected | - | undetected |
| Example 5 | 3HALE | 12.8-12.9 | methanol/ water | 10 | 100 | 50 | 28 | 6 | - | 4 |
| Example 6 | 3HALE | 8.6-11.0 | methanol/ water | 10 | 100 | 78 | 0.1 | 2.6 | - | undetected |
| Example 7 | 3HALE | 12.3-12.5 | methanol | 0 | 100 | 93 | 5 | 2 | - | undetected |
| Example 8 | 3HALE | 11.0-12.2 | methanol/ water | 50 | 100 | 51 | 32 | undetected | - | 18 |
| Example 9 | 3HALE | 10.5-12.3 | methanol/ water | 70 | 100 | 46 | 18 | 2 | - | 34 |
| Example 10 | 3HALE | 9.2-12.2 | methanol/ water | 90 | 100 | 31 | 8 | 2 | - | 50 |
| Example 11 | 3HALE | 10.3-10.6 | acetone/ water | 50 | 85 | 59 | 0.2 | undetected | - | undetected |
| Example 12 | 3HALE | 12.5-12.9 | acetone/ methanol | 0 | 87 | 68 | 0.7 | 22 | - | 1.9 |
| Comparative Example 1 | 3HALE | 13.0-13.3 | methanol/ water | 10 | 100 | 2 | 55 | undetected | - | 4 |
| Comparative Example 2 | 3HALE | 7.6-8.0 | methanol/ water | 10 | 11 | 6.9 | undetected | 93 | - | undetected |

(continued)

| Reaction | Starting material | pH range | Solvent | Proportion of water to reaction solvent (vol%) | Conversion of starting material (%) | Product Selectivity (%) | | | | |
|---|---|---|---|---|---|---|---|---|---|---|
| | | | | | | HMAM | HMA | 3HAD | 3HALE | 3HA |
| Comparative Example 3 | 3HALE | 14.0-14.2 | acetone/water | 50 | 100 | undetected | 73 | undetected | - | 3.8 |

HMAM: monomethyl α-hydromuconate (III-1), HMA: α-hydromuconic acid, 3HAD: dimethyl 3-hydroxyadipate (I-1), 3HALE: 3-hydroxyadipic acid-3,6-lactone methyl ester (II-1), 3HA: 3-hydroxyadipic acid

Examples 1 to 12 demonstrated that an α,β-unsaturated carboxylic acid ester represented by general formula (III) can be selectively produced by subjecting either any of carboxylic acid esters each represented by general formula (I) or (II) or a mixture of two or more thereof to basic conditions with a pH of 8.5 or higher but less than 13 in an organic solvent or in a mixed solvent including an organic solvent and water. Example 10 gave results indicating high selectivity to 3-hydroxyadipic acid. However, since 3-hydroxyadipic acid can be easily converted to an α,β-unsaturated dicarboxylic acid ester as shown in Example 14, which will be given later, Example 10 was regarded as substantially having high selectivity to the α,β-unsaturated carboxylic acid ester. Furthermore, Example 11 demonstrated that similar results are obtained also in a mixed solvent composed of an organic solvent other than methanol and water, and Example 12 demonstrated that an α,β-unsaturated dicarboxylic acid ester can be selectively produced also in a mixed solvent composed of several organic solvents.

Meanwhile, Comparative Examples 1 to 3 showed that in cases when the reaction solution has a pH of 13 or higher or less than 8.5, the selectivity to an α,β-unsaturated dicarboxylic acid ester is considerably low; in Comparative Example 3, no α,β-unsaturated dicarboxylic acid ester was obtained.

(Example 13) Production of Dimethyl α-Hydromuconate (III-10) from Monomethyl α-Hydromuconate (III-1)

One milligram of monomethyl α-hydromuconate and 0.9 mL of methanol (manufactured by FUJIFILM Wako Pure Chemical Corp.) were used and introduced into a vial made of glass having a capacity of 2 mL (manufactured by LABORAN), and 0.1 mL of 1-M aqueous sulfuric acid solution (manufactured by Nacalai Tesque, Inc.) was added as a catalyst thereto. The reaction solution was refluxed for 6 hours with stirring at 300 rpm and then recovered. A 0.1-mL portion of the reaction solution was diluted with 0.9 mL of water, filtered with a 0.22-μm filter, and then analyzed by HPLC. Yield of dimethyl α-hydromuconate was 94%.

This Example demonstrated that an α,β-unsaturated dicarboxylic acid diester can be produced by esterifying an α,β-unsaturated dicarboxylic acid monoester which can be produced by the present invention.

(Example 14) Production of Dimethyl α-Hydromuconate (III-10) from 3-Hydroxyadipic Acid as Starting Material

A hundred milligrams of 3-hydroxyadipic acid and 9 mL of methanol (manufactured by FUJIFILM Wako Pure Chemical Corp.) were used and introduced into a round-bottom flask having a capacity of 25 mL (manufactured by IWAKI, AGC TECHNO GLASS Co., Ltd.), and one drop of concentrated sulfuric acid was added thereto. This reaction solution was refluxed for 3 hours with stirring at 300 rpm. One milliliter of 0.5-M aqueous sodium hydrogen carbonate solution was added to 9 mL of the obtained methanol solution, which contained 70 mg of dimethyl 3-hydroxyadipate (1-1) and 49 mg of 3-hydroxyadipic acid-3,6-lactone methyl ester (II-1). The used 0.5-M aqueous sodium hydrogen carbonate solution was one obtained by dissolving 0.42 g of sodium hydrogen carbonate (manufactured by Nacalai Tesque, Inc.) in 10 mL of water. The pH of the reaction solution during the reaction was 11.0-12.5. The reaction solution was refluxed for 3 hours with stirring at 300 rpm. The obtained reaction solution, which contained 96 mg of monomethyl α-hydromuconate (III-1), was returned to ambient temperature, and 10 drops of concentrated sulfuric acid were added thereto to adjust the pH of the reaction solution to 1 or less. This reaction solution was reacted at ambient temperature for 72 hours with stirring at 300 rpm. A 0.1-mL portion of the reaction solution was diluted with 0.9 mL of water, filtered with a 0.22-μm filter, and then analyzed by HPLC. Yield of dimethyl α-hydromuconate was 68%.

(Example 15) Production of Dimethyl α-Hydromuconate (III-10) from 3-Hydroxyadipic acid-3,6-lactone as Starting Material

Reactions were conducted in the same manner as in Example 14, except that 3-hydroxyadipic acid-3,6-lactone was used as a starting material in place of the 3-hydroxyadipic acid. Yield of dimethyl α-hydromuconate was 70%.

(Example 16) Recovery of Dimethyl α-Hydromuconate (III-10)

The solution obtained in Example 14 which contained dimethyl α-hydromuconate was concentrated with a rotary evaporator and then distilled at 80°C at a reduced pressure of 800 Pa, thereby obtaining 59 mg of pure dimethyl α-hydromuconate. Yield through the distillation was 82%.

Examples 14 and 15 demonstrated that dimethyl α-hydromuconate (III-10) can be produced by esterifying either 3-hydroxyadipic acid, which can be produced by either chemical synthesis or microbial fermentation, or 3-hydroxyadipic acid-3,6-lactone, which can be easily produced by treating 3-hydroxyadipic acid with an acid, to thereby yield a mixture of dimethyl 3-hydroxyadipate (1-1) and 3-hydroxyadipic acid-3,6-lactone methyl ester (II-1), subsequently subjecting these esters to basic conditions with a pH of 8.5 or higher but less than 13 to thereby yield monomethyl α-hydromuconate (III-1), and then esterifying the monomethyl α-hydromuconate. Furthermore, Example 16 showed that the dimethyl α-hydromuconate (III-10) can be recovered by distillation.

## Claims

1. A method of producing an α,β-unsaturated dicarboxylic acid ester represented by general formula (III), the method comprising a step in which one or more carboxylic acid esters represented by general formula (I) and/or general formula (II) are subjected to a basic condition with pH of 8.5 or higher but less than 13 in either an organic solvent or a mixed solvent comprising an organic solvent and water:

[Chem. 1]

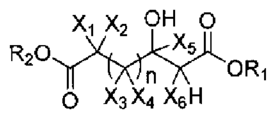
(I)

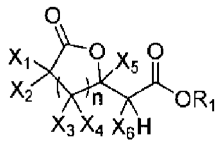
(II)

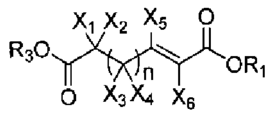
(III)

wherein, n is an integer of 1-3, $X_1$ to $X_6$ each independently represent a hydrogen atom (H), an alkyl group having 1-6 carbon atoms, or a phenyl group, $R_1$ and $R_2$ each independently represent an alkyl group having 1-6 carbon atoms, and $R_3$ represents a hydrogen atom (H) or an alkyl group having 1-6 carbon atoms.

2. The method according to claim 1, wherein the organic solvent is a water-miscible organic solvent.

3. The method according to claim 1 or 2, wherein the mixed solvent comprising an organic solvent and water has a water content of 90% by volume or less.

4. The method according to any one of claims 1 to 3, wherein the carboxylic acid ester represented by general formula (I) is a 3-hydroxyadipic acid ester.

5. The method according to any one of claims 1 to 3, wherein the carboxylic acid ester represented by general formula (II) is a 3-hydroxyadipic acid-3,6-lactone ester.

## INTERNATIONAL SEARCH REPORT

International application No.
PCT/JP2020/007286

A. CLASSIFICATION OF SUBJECT MATTER
C07C 69/593(2006.01)i; C07C 67/30(2006.01)i; C07C 67/327(2006.01)i
FI: C07C67/327; C07C67/30; C07C69/593
According to International Patent Classification (IPC) or to both national classification and IPC

B. FIELDS SEARCHED
Minimum documentation searched (classification system followed by classification symbols)
C07C69/593; C07C67/30; C07C67/327

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>A | PINHEIRO, S. et al., "Piperonal as starting material for the regiospecific and stereoselective synthesis of E and Z isomers of 3-methyl-2-hexenedioic acid 1-methyl ester", Synthetic Communications, 1991, vol. 21, no. 5, pp. 703-12 pp. 703-705, scheme 1, table 1 | 1-2, 5<br>3-4 |
| X<br>A | LI, Z. et al., "Copper-catalyzed cascade reactions of α, β-unsaturated esters with keto esters", Beilstein Journal of Organic Chemistry, 2015, vol. 11, pp. 213-218 p. 216, fig. 3 | 1<br>2-5 |
| A | JP 2003-522812 A (BASF AG.) 29.07.2003 (2003-07-29) entire text, all drawings, in particular, claims, examples | 1-5 |
| A | WO 2016/068108 A1 (TORAY INDUSTRIES, INC.) 06.05.2016 (2016-05-06) entire text, all drawings, in particular, claims, paragraphs [0025]-[0026], examples | 1-5 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 April 2020 (22.04.2020) | 12 May 2020 (12.05.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/007286

| Patent Documents referred in the Report | Publication Date | Patent Family | Publication Date |
|---|---|---|---|
| JP 2003-522812 A | 29 Jul. 2003 | US 2003/0013906 A1 entire text, all drawings, in particular, claims, examples<br>EP 1255722 A1 | |
| WO 2016/068108 A1 | 06 May 2016 | US 2017/0320819 A1 entire text, all drawings, in particular, claims, paragraphs [0025]-[0026], examples<br>EP 3214072 A1<br>CN 107074761 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015187129 A
- WO 2016199856 A
- WO 2016068108 A


### Non-patent literature cited in the description


- *Journal of Chemical Society,* 1956, 4426-4428
- *Journal of Chemical Society C: Organic,* 1967, vol. 22, 2314-2316